# EUROPEAN PATENT SPECIFICATION

(11) **EP 2 827 881 B1**
(45) Date of publication and mention of the grant of the patent: **22.06.2016**
(21) Application number: 13721088.6
(22) Date of filing: 21.03.2013
(51) Int. Cl.: A61K 36/886, A61K 36/22, A61P 17/00

(54) **RHUS CORIARIA L. EXTRACT FOR USE IN THE TREATMENT OF HYPERHIDROSIS**
EXTRAKT AUS RHUS CORIARIA L. ZUR VERWENDUNG BEI DER BEHANDLUNG VON HYPERHIDROSE
EXTRAIT DE RHUS CORIARIA L DESTINÉ À ÊTRE UTILISÉ DANS LE TRAITEMENT DE L'HYPERHIDROSE

(30) Priority: 23.03.2012 IT MI20120462
(43) Date of publication of application: 28.01.2015
(73) Proprietor: Biomedical Research S.r.l., 95128 Catania (IT)
(72) Inventor: ZARBO, Giuseppa, I-95128 Catania (IT)
(74) Representative: Aprà, Mario
(86) International application number: PCT/IB2013/052243
(87) International publication number: WO 2013/140361

(56) References cited:
- WO-A2-03/101389
- DE-A1-102008 037 633
- FR-A1- 2 814 075
- FERK FRANZISKA ET AL: "Antioxidant and free radical scavenging activities of sumac (Rhus coriaria) and identification of gallic acid as its active principle", BMC PHARMACOLOGY, BIOMED CENTRAL, LONDON, GB, vol. 7, no. Suppl 2, 14 November 2007 (2007-11-14), page A71, XP021028729, ISSN: 1471-2210, DOI: 10.1186/1471-2210-7-S2-A71
- KOSAR ET AL: "Antioxidant activity and phenolic composition of sumac (Rhus coriaria L.) extracts", FOOD CHEMISTRY, ELSEVIER LTD, NL, vol. 103, no. 3, 24 February 2007 (2007-02-24), pages 952-959, XP022345098, ISSN: 0308-8146, DOI: 10.1016/J.FOODCHEM.2006.09.049
- SEMSETTIN KARACA ET AL: "Erythrocyte Oxidant/Antioxidant Status in Essential Hyperhidrosis", MOLECULAR AND CELLULAR BIOCHEMISTRY, KLUWER ACADEMIC PUBLISHERS, BO, vol. 290, no. 1-2, 23 May 2006 (2006-05-23), pages 131-135, XP019436637, ISSN: 1573-4919, DOI: 10.1007/S11010-006-9177-8
- NAZYROVA R M ET AL: "Non-alcoholic fizzy drink - contains sugar, citric acid, aq. alcoholic infusions of specified berries and currant leaves and carbonated water", WPI / THOMSON,, vol. 1983, no. 5, 30 March 1982 (1982-03-30), XP002663475,

## Description

The present invention relates to extracts of *Rhus coriaria* L. for use in the treatment of hyperhidrosis in humans.

### Prior art

The human body possesses over four million sweat glands, divided into two types: the eccrine glands, which are responsible for the physiological function of regulating the body temperature and eliminating toxins, but are also stimulated by many other circumstances, for example in situations of fear, anger, sadness, physical pain, embarrassment, stress, physical effort and emotional difficulties, and the apocrine glands, which only react to stimuli originating from the involuntary nervous system and the hormones.

The sweat manufactured by these glands is odourless in itself. The problem arises when it comes into contact with the bacteria present on the skin, breaks down and forms new substances, which often have an unpleasant odour.

Unlike other warm-blooded living organisms, humans have glands all over the body, but not equally distributed. They are located mainly in the hands, feet, forehead and underarms; for example, there are over 25,000 apocrine and eccrine glands in the underarms.

Sweating becomes excessive when (very rarely) the apocrine glands are over-stimulated (in the event of hormonal dysfunctions or dysfunctions of the nervous system) or (very frequently) when the eccrine glands are over-stimulated by fear, heat, sadness, anger, stress, physical effort or emotional problems. This dysfunction is called "hyperhidrosis", and up to 20% of the adult population suffer from it.

Hyperhidrosis is therefore defined as sweating in excess of the amount necessary for normal body temperature regulation. Hyperhidrosis is classed as localised (or focal) when the excessive sweating is limited to one or a few parts of the body, and as generalised when it affects the entire body. Hyperhidrosis can become a disabling disorder, especially when it affects the hands or underarms, but hyperhidrosis of the face, head, torso, groin and feet can also cause great discomfort.

The condition is classed as primary or idiopathic hyperhidrosis when no cause for the excessive sweating can be identified: this disorder is believed to be due to excessive ("hypertonic") activity of the sympathetic nervous system. Although some patients report that they have suffered from it since childhood, excess sweating usually begins in adolescence and continues into adulthood, while the symptoms tend to attenuate in old age. Hyperhidrosis is slightly more frequent in the female than the male gender. A genetic predisposition is probable, as 40-50% of patients have a family history of hyperhidrosis.

Secondary hyperhidrosis is a consequence of other disorders, such as obesity or the menopause, or arises as a result of endocrine, metabolic, neurological, neoplastic or cardiorespiratory disorders, and is sometimes due to serious infections or is a side effect of some drugs. It mainly affects elderly people. In these cases, the sweating usually affects the whole body, and the signs and symptoms of the underlying disorder are also present.

The main diagnostic criterion that enables primary hyperhidrosis to be identified is excessive, limited, visible sweating lasting for at least 6 months, with no apparent cause, which has at least 2 of the following characteristics:
- bilateral, relatively symmetrical, sweating,
- at least one episode a week,
- hindrance to the patient's everyday activities,
- positive family history,
- cessation of sweating during sleep.

For a long time it was erroneously believed that hyperhidrosis was caused by anxiety, whereas the opposite is the case: hyperhidrosis creates anxiety (which further worsens the symptoms). Primary localised (or focal) hyperhidrosis becomes a problem from adolescence, when sweating causes the first embarrassments in social life. The sites of hyperhidrosis which have the greatest emotional consequences are the palms of the hands (often in association with the soles of the feet), the underarms and the face.

A clinical sign characteristic of palmar hyperhidrosis is the simultaneous presence of sweating and vasoconstriction, which leads to the typical cold, perspiring hand that is so unpleasant during a handshake. In some patients, sweating declines during the winter, but reappears in situations of stress, and worsens in summer. The quantity of sweat can vary considerably: the palm of the hand may be slightly damp in some patients but drip with sweat in others, so that shaking hands can become an insurmountable obstacle, and even talking on a mobile phone or typing on a computer can be unpleasant.

Underarm sweating can be so abundant that patients are forced to change their clothes several times a day, and only wear black or white clothes to conceal the problem; patients who suffer from hyperhidrosis of the head or face know that they can start sweating, and even dripping with sweat, without any specific reason, perhaps while driving, watching television or eating.

Hyperhidrosis often leads to physical disorders, such as bacterial or fungal infections caused by maceration of the skin, or mental disorders, such as difficulty with social and personal relationships, frustration in everyday activities, changes in the usual type of leisure activities, lack of confidence with other people, low self-esteem and depression.

The treatment for secondary hyperhidrosis is medical, and is designed to treat the underlying disorder that causes the excessive sweating; for example, weight reduction in the case of obesity, and suitable hormone treatment in the case of hyperthyroidism.

The treatment of primary localised hyperhidrosis can be medical or surgical, but even nowadays the results are not wholly satisfactory. Antiperspirant creams containing aluminium chloride, or iontophoresis, can slightly reduce the sweating, but only in the case of mild hyperhidrosis. In some patients, results have been obtained with *Clostridium botulinum* toxin. The botulinum toxin acts by blocking the release of acetylcholine at the nerve synapses of the neuromuscular joints of the sweat glands, leading to a reduction in sweating. This procedure, which is painful and not risk-free, involves multiple injections of toxin into the sites concerned (hands, underarms, etc.) with a thin needle. The injections must be performed accurately into the dermis to avoid side effects, especially loss of strength if the hand muscles are accidentally punctured. Moreover, the efficacy of the injections does not exceed 5-6 months, so the treatment has to be repeated at least 2-3 times a year.

Surgery is used to treat primary hyperhidrosis of the hands, underarms, head and face. It is performed with a minimally invasive thoracic endoscopy technique known as videothoracoscopic sympathectomy (VTS), which acts selectively on the ganglia of the thoracic sympathetic nervous system, which are responsible for controlling the sweat glands of the hands, underarms, head and face.

Two small incisions (approx. 5-6 millimetres) are made in the underarm cavity under general anaesthetic; the videothoracoscope is introduced through the first port, and the chain of nerve ganglia running laterally and parallel to the spinal column, beneath the parietal pleura, on the rear costal section, is identified. The surgical instrument used to perform the operation on the sympathetic chain ganglia is introduced through the second port.

This is to all effects a surgical operation performed under general anaesthetic; a specialist examination by the thoracic surgeon is required before the patient is admitted to hospital, and the possible appearance of a severe side effect that can be caused by the surgery (compensatory hyperhidrosis) must always be taken into account.

### Description of the invention

It has now been found that extracts of *Rhus coriaria* L. are able to treat hyperhidrosis in an entirely natural way, by regulating the secretion of both types of sweat gland, reducing excessive sweating and restoring the normal perspiration required to ensure correct body temperature regulation.

The antioxidant activity of *Rhus coriaria* is known to decline over time due to a decrease in polyphenols. See, for example, Ozcan M. (2003) Effect of sumach (Rhus coriaria L.) extracts on the oxidative stability of peanut oil. J Med Food 6(1):63-6.

The activity observed for the compositions according to the invention against hyperhidrosis is therefore not attributable to antioxidant effects. Moreover, numerous other natural substances with marked antioxidant activity have no therapeutic effect on the disorder in question.

The invention therefore provides compositions containing an extract of *Rhus coriaria* L. as active ingredient for use in the treatment of hyperhidrosis.

The advantageous, preferred method of preparation of the extract is steam distillation (hydrolates, essential oils, aromatic waters), but a useful active ingredient can also be obtained by non-distillative extraction processes (infusion, decoction, digestion, maceration, etc.).

The extracts can be formulated, using conventional techniques and excipients, in the form of sprays, creams, ointments, salves or other forms suitable for topical administration, or can be used to prepare medical devices, hydrogels, sticking plasters, transdermal patches, armpit pads and foot pads. Said compositions according to the invention will typically contain 0.5 to 20% by weight of *Rhus coriaria* L. extract.

The dose and administration regimen can be determined by the doctor on the basis of the patient's condition and the severity of the hyperhidrosis. Broadly speaking, as reported in the clinical trials specified below by way of example, one application of a spray or cream containing *Rhus coriaria* L. extract in the morning, preceded by simple washing with soap and water, and one application of a cleansing product containing the same extract in the evening, may be sufficient.

According to the results of a study performed on 30 volunteers suffering from serious forms of primary hyperhidrosis, it is sufficient to apply said extract to the affected parts (localised hyperhidrosis), or use it all over the body as a liquid composition (generalised hyperhidrosis), to obtain better results than conventional medical or surgical treatment.

The results of the various medical treatments currently used for hyperhidrosis (antiperspirant creams containing aluminium chloride, iontophoresis, and botulinum toxin), were compared with daily application of products containing a *Rhus coriaria* L extract.

Mediocre results were obtained with the conventional methods, and only against mild forms of hyperhidrosis, whereas the use of products containing *Rhus coriaria* L. extract neutralised excessive sweating, even in the more serious and lengthy forms of hyperhidrosis, at the same time maintaining the normal perspiration necessary to ensure correct body temperature regulation, and preventing the formation of unpleasant odours.

The use of products containing *Rhus coriaria* L. extract to treat hyperhidrosis even proved superior to surgical treatment, and has none of its side effects (compensatory hyperhidrosis).

As hyperhidrosis can be considered a multifactorial disorder, characterised not only by excessive sweating but also by skin maceration, bacterial or fungal infections and formation of unpleasant odours, it has been found that the combination of extracts of *Rhus coriaria* L. and *Aloe barbadensis* Miller, better known as Aloe vera, has a synergic effect against hyperhidrosis, accentuating the therapeutic effects of *Rhus coriaria* L extracts. This synergy is definitely attributable to the well-known moisturising, anti-inflammatory, soothing, refreshing, bactericidal and antifungal properties of *Aloe barbadensis* Miller extracts (gel, juice, cream), which enhance the activities of *Rhus coriaria* L. extracts against hyperhidrosis. It is sufficient to add about 20% to 40% by weight of an *Aloe barbadensis* Miller extract in one of the forms reported above (gel, juice or cream), depending on the type of product made with the *Rhus coriaria* L. extract (spray, cream, ointment, salve, etc.), to enhance the curative effect of the latter on hyperhidrosis.

These results demonstrate that treatment of hyperhidrosis with *Rhus coriaria* L. extract, used alone or in combination with *Aloe barbadensis* Miller, extracts, is more effective than the medical and surgical treatments currently used, and represents a novel, economical, non-invasive, painless technique for the treatment of this disorder, with no side effects.

## Claims

1. Compositions comprising as active ingredient an extract of *Rhus coriaria* L., optionally in combination with extracts of *Aloe barbadensis* Miller, for use in the treatment of hyperhidrosis.

2. Compositions for use according to claim 1 in liquid form or as sprays, creams, salves or ointments.

3. Compositions for use according to claim 1 in the form of medical devices, hydrogels, patches, transdermal patches, armpit pads and foot pads.

## Patentansprüche

1. Zusammensetzungen, die als Wirkstoff ein Extrakt aus *Rhus coriaria* L., optional in Verbindung mit Extrakten aus *Aloe barbadensis* Miller, umfassen, zur Verwendung bei der Behandlung von Hyperhidrose.

2. Zusammensetzungen zur Verwendung nach Anspruch 1 in flüssiger Form oder als Sprays, Cremes, Pasten oder Salben.

3. Zusammensetzungen zur Verwendung nach Anspruch 1 in Form von Medizinprodukten, Hydrogelen, Pflastern, Transdermalpflastern, Achselpads und Fußpads.

## Revendications

1. Compositions comprenant en tant qu'ingrédient actif un extrait de *Rhus coriaria* L., facultativement en combinaison avec des extraits d'*Aloe barbadensis* Miller, destinées à être utilisées dans le traitement de l'hyperhidrose.

2. Compositions destinées à être utilisées selon la revendication 1 sous forme liquide ou en tant que sprays, crèmes, pommades ou onguents.

3. Compositions destinées à être utilisées selon la revendication 1 sous la forme de dispositifs médicaux, d'hydrogels, de patchs, de timbres transdermiques, de tampons pour aisselle et de tampons pour pied.
